# EUROPÄISCHE PATENTSCHRIFT

(11) **EP 0 695 738 B1**
(45) Veröffentlichungstag und Bekanntmachung des Hinweises auf die Patenterteilung: **28.05.1997**
(21) Anmeldenummer: 95111896.7
(22) Anmeldetag: 28.07.1995
(51) Int. Cl.: C07C 231/02, C07C 235/16

(54) **Verfahren zur Herstellung von O-Acylglykolsäureaniliden**
Process for the preparation of 0-acylglycollic acid anilides
Procédé pour la préparation d'anilides de l'acide 0-acyl-glycollique

(30) Priorität: 05.08.1994 DE 4427837
(43) Veröffentlichungstag der Anmeldung: 07.02.1996
(73) Patentinhaber: HOECHST AKTIENGESELLSCHAFT, 65929 Frankfurt am Main (DE)
(72) Erfinder: Dierdorf, Andreas, Dr., D-60529 Frankfurt am Main (DE); Papenfuhs, Theodor, Dr., D-60433 Frankfurt am Main (DE)

(56) Entgegenhaltungen:
- EP-A- 0 005 501
- WO-A-93/22278
- DE-A- 3 038 598
- DATABASE WPI Week 7727 Derwent Publications Ltd., London, GB; AN 77-47608Y & JP-A-52 062 234 (KUMIAI CHEM IND KK) , 23.Mai 1977
- DATABASE WPI Week 7809 Derwent Publications Ltd., London, GB; AN 78-16369A & JP-A-50 148 321 (KUMIAI CHEM IND KK) , 27.November 1975

## Beschreibung

Die vorliegende Erfindung betrifft ein gegenüber dem Stand der Technik verbessertes Verfahren zur Herstellung von O-Acylglykolsäureaniliden.

O-Acylglykolsäureanilide, insbesondere Acetoxyessigsäureanilide, stellen interessante Vorprodukte zur Herstellung von Glykolsäureaniliden dar. Glykolsäureanilide wiederum finden als wichtige Ausgangsstoffe sowohl bei der Herstellung von Herbiziden (EP-A-300 344) und pharmazeutischen Wirkstoffen (EP-A-284 338, EP-A-363 284) als auch bei der Herstellung von Fungiziden (US 4 440 780) Anwendung.

Aufgrund der Bedeutung dieser Stoffgruppe hat es in der Vergangenheit nicht an Versuchen gefehlt, Hvdroxycarbonsäureamide und insbesondere Hvdroxycarbonsäureanilide, beispielsweise Glykolsäureanilide, auf unterschiedlichen Wegen zugänglich zu machen.

So geht aus der DE-OS 30 38 598 ein Verfahren zur Herstellung von α-Hydroxycarbonsäureamiden durch Umsetzung von α-Acyloxycarbonsäureamiden (O-acylierte α-Hydroxycarbonsäureamide), insbesondere von α-Formyloxycarbonsäureamiden, mit Alkylalkoholen in Anwesenheit katalytischer Mengen von Alkali- oder Erdalkali-Hydroxiden, Hydrogencarbonaten oder Carbonaten hervor. Die entsprechenden α-Acyloxycarbonsäureamide erhält man, indem man α-Chlorcarbonsäureamide mit Alkalimetallformiaten oder -acetaten umsetzt. Da die für diese Umsetzung benötigten α-Chlorcarbonsäureamide in einem separaten Schritt, beispielsweise durch Umsetzung eines Amins mit dem entsprechenden α-Chlor-carbonsäurechlorid, hergestellt werden müssen, handelt es sich bei der Herstellung der α-Oxycarbonsäureamide in Wirklichkeit um ein zweistufiges Verfahren, das zudem noch den Nachteil aufweist, daß die Herstellung der α-Oxycarbonsäureamide in Gegenwart eines quartären Ammoniumsalzes durchgeführt wird. Es ist jedoch bekannt, daß derartige quartäre Ammoniumsalze zu Problemen in der Abwasseraufarbeitung führen.

Ein weiteres Verfahren zur Herstellung von α-Hydroxycarbonsäureamiden durch Umesterung von Acetoxycarbonsäureamiden mittels Alkoholen in Gegenwart katalytischer Mengen eines Alkali- oder Erdalkalihydroxids oder Alkali- oder Erdalkalicarbonats ist der DE-OS 29 04 490 zu entnehmen. Man setzt die entsprechenden α-Halogencarbonsäureamide mit einem Alkali- oder Erdalkaliacetat in Gegenwart eines quartären Ammoniumsalzes und gegebenenfalls unter Verwendung eines Verdünnungsmittels zu den entsprechenden α-Acetoxycarbonsäureamiden um.

Da die hierfür benötigten α-Chlorcarbonsäureamide in einem separaten Schritt, beispielsweise durch Umsetzung eines Amins mit dem entsprechenden α-Chlorcarbonsäurechlorid, hergestellt werden müssen, handelt es sich bei der Herstellung der α-Acetoxycarbonsäureamide ebenfalls um ein zweistufiges Verfahren, bei dem die Verwendung quartärer Ammoniumsalze ebenfalls zu einer unerwünschten Belastung des Abwassers führt.

Die US 4 509 971 beschreibt in Spalte 6, Zeile 41 bis Spalte 7, Zeile 16 und Spalte 26, Zeilen 14 bis 56 ein Verfahren zur Herstellung von Acetoxyessigsäureamiden und insbesondere Acetoxyessigsäureaniliden, das von Hydroxyessigsäure ausgeht. Man setzt zunächst in einem separaten Schritt Hydroxyessigsäure mit Acetylchlorid um und erhält Acetoxyessigsäure. In einem weiteren Schritt wird die Acetoxyessigsäure direkt mit Thionylchlorid zu Acetoxyessigsäurechlorid umgesetzt, das anschließend isoliert wird. Durch Umsetzung des Acetoxyessigsäurechlorids mit einem sekundären Amin oder Anilin erhält man die entsprechenden Acetoxyessigsäureamide oder Acetoxyessigsäureanilide.

Bezogen auf Acetoxyessigsäure als Ausgangsmaterial sind für die Herstellung der entsprechenden Acetoxyessigsäureamide oder Acetoxyessigsäureanilide also zwei Schritte erforderlich, nämlich erstens die Umsetzung von Acetoxyessigsäure mit Thionylchlorid, Isolierung des Acetoxyessigsäurechlorids und zweitens die Umsetzung des Acetoxyessigsäurechlorids mit dem sekundären Amin oder Anilin.

Die vorstehend geschilderten Verfahren erfordern einen relativ großen Aufwand, da sie die gewünschten α-Acetoxyessigsäureamide (O-Acetylglykolsäureamide) über zwei separate, nacheinander ablaufende Reaktionsschritte zugänglich machen. Zudem führen die als Phasentransfer-Katalysatoren verwendeten quartären Ammoniumsalze zu Problemen bei den im Verlauf der Umsetzung anfallenden Abfallprodukten. Sie sind insbesondere aufgrund ihrer ungünstigen Eigenschaften im Abwasser unerwünscht.

Im Hinblick auf die Bedeutung von O-Acylglykolsäureaniliden als Vorprodukte zur Herstellung von Glykolsäureaniliden stellt es eine interessante Aufgabe dar, ein Verfahren zur Herstellung von O-Acylglykolsäureaniliden bereitzustellen, das einerseits die Nachteile der vorstehend genannten Verfahren vermeidet, sich andererseits auf einfache Weise und unter Verwendung leicht zugänglicher Ausgangsstoffe durchführen läßt und zudem die gewünschten O-Acylglykolsäureanilide in guter Ausbeute und hoher Reinheit liefert.

Gelöst wird diese Aufgabe durch ein Verfahren zur Herstellung von O-Acylglykolsäureaniliden der allgemeinen Formel (1) 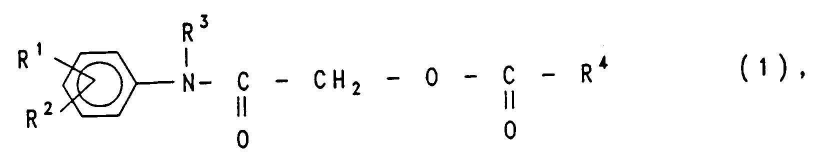 worin R¹ und R² gleich oder verschieden sind und für Wasserstoff, Halogen, eine Nitro-, Cyano-, eine geradkettige oder verzweigte Alkyl-, Alkenyl-, Alkinyl-, Alkoxy-Gruppe mit 1 bis 12 Kohlenstoffatomen, eine Aralkyl-Gruppe mit 7 bis 12 Kohlenstoffatomen, eine Cycloalkyl-Gruppe mit 6 bis 12 Kohlenstoffatomen oder eine Aryl-Gruppe mit 6 bis 12 Kohlenstoffatomen stehen, R³ Wasserstoff oder eine geradkettige oder verzweigte Alkyl-Gruppe mit 1 bis 12 Kohlenstoffatomen bedeutet und R⁴ für eine Alkyl-Gruppe mit 1 bis 6 Kohlenstoffatomen oder einen substituierten oder unsubstituierten Phenylrest steht. Es ist dadurch gekennzeichnet, daß man ein Anilin der allgemeinen Formel (2) 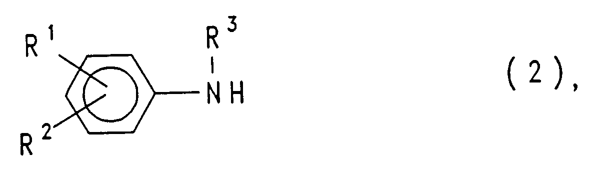 worin R¹, R² und R³ dieselbe Bedeutung wie in Formel (1) haben und eine O-Acylglykolsäure der allgemeinen Formel (3) 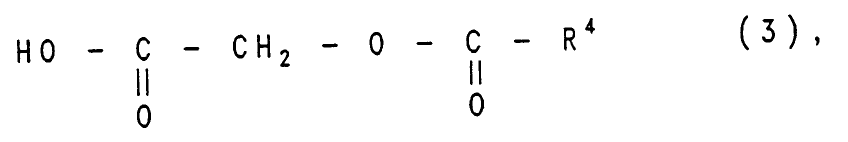 worin R⁴ dieselbe Bedeutung wie in Formel (1) hat, mit einem anorganischen Säurehalogenid, gegebenenfalls in Anwesenheit eines inerten organischen Lösungsmittels, bei 20 bis 180° C umsetzt.

Das erfindungsgemäße Verfahren weist mehrere Vorteile auf. Zum einen führt es - wie die nachfolgende Reaktionsgleichung unter Verwendung von PCl₃ als anorganisches Säurehalogenid beispielhaft belegt - in einer einzigen Reaktionsstufe zu den gewünschten O-Acylglykolsäureaniliden.

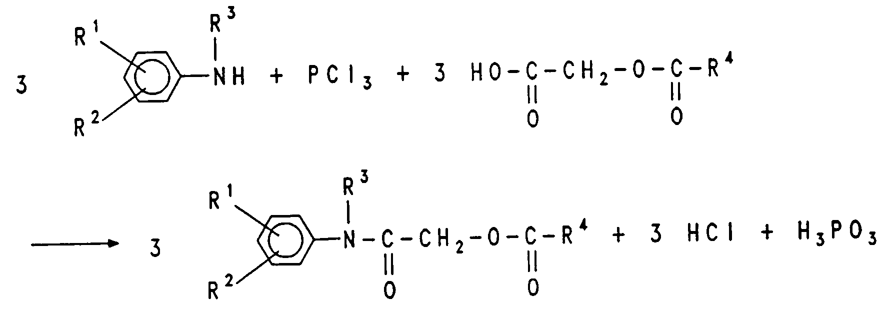

Wie der Reaktionsgleichung zu entnehmen ist, werden das Anilin der Formel (2), die O-Acylglykolsäure der Formel (3) und das anorganische Säurechlorid gleichzeitig miteinander zur Reaktion gebracht und die gewünschten O-Acylglykolsäureanilide der Formel (1) bilden sich unmittelbar.

Zum anderen ist es daher nicht erforderlich, auf aufwendige Art und Weise irgendein Zwischenprodukt zu isolieren, um es anschließend weiterzuverarbeiten. Zum anderen kann auf die Verwendung von quartären Ammoniumsalzen als Phasentransferkatalysatoren generell verzichtet werden. Das erfindungsgemäße Verfahren läßt sich darüber hinaus ohne großen technischen Aufwand und unter Verwendung leicht zugänglicher Ausgangsprodukte realisieren. Die gewünschten O-Acylglykolsäureanilide fallen in guter Ausbeute und zugleich sehr hoher Reinheit an.

Das gegebenenfalls in die Reaktion eingesetzte inerte organische Lösungsmittel läßt sich nach Beendigung der Umsetzung destillativ abtrennen und kann, falls gewünscht, wieder in die Reaktion eingesetzt werden.

Wie aus den vorangegangenen Ausführungen hervorgeht, eignet sich das erfindungsgemäße Verfahren für die Umsetzung einer größeren Zahl verschiedener Aniline. Insbesondere lassen sich Aniline der Formel (2), worin R¹ und R² gleich oder verschieden sind und für Wasserstoff, Halogen, eine Nitro-, eine geradkettige oder verzweigte Alkyl-, Alkoxy-Gruppe mit 1 bis 4 Kohlenstoffatomen, oder eine Aralkyl-Gruppe mit 7 bis 12 Kohlenstoffatomen, insbesondere für Wasserstoff, Fluor, Chlor, Brom, eine Alkyl- oder Alkoxy-Gruppe mit 1 bis 4 Kohlenstoffatomen, bevorzugt für Wasserstoff, Fluor, Chlor, Brom oder eine Alkylgruppe mit 1 bis 4 Kohlenstoffatomen stehen, einsetzen.

Wie eingangs bereits erwähnt, setzt man ein Anilin der Formel (2), worin R³ Wasserstoff oder eine geradkettige oder verzweigte Alkylgruppe mit 1 bis 12 Kohlenstoffatomen bedeutet, insbesondere ein Anilin der Formel (2), worin R³ für Wasserstoff oder eine geradkettige oder verzweigte Alkylgruppe mit 1 bis 4 Kohlenstoffatomen steht, bevorzugt ein Anilin der Formel (2), worin R³ für eine Isopropylgruppe steht, ein.

Beispiele für einige geeignete Aniline sind, ohne Anspruch auf Vollständigkeit zu erheben, 2-Methoxyanilin, 4-Methoxyanilin, 3,5-Dimethylanilin, 2-Chloranilin, 4-Chloranilin, N-Methylanilin, N-Ethylanilin, N-Isopropylanilin und N-Isopropyl-4-fluoranilin.

Man setzt in die Reaktion eine O-Acylglykolsäure der allgemeinen Formel (3), worin R⁴ für eine Alkyl-Gruppe mit 1 bis 6 Kohlenstoffatomen oder einen gegebenenfalls substituierten Phenylrest, insbesondere für eine Alkyl-Gruppe mit 1 bis 4 Kohlenstoffatomen, beispielsweise für eine Methyl-, Ethyl-, n-Propyl-, i-Propyl-Gruppe, oder einen Phenylrest, bevorzugt für eine Methyl-Gruppe steht, ein.

Beispiele für O-Acylglykolsäuren sind Acetoxyessigsäure, O-Propionylglykolsäure, O-n-Butyrylglykolsäure, O-Isobutyrylglykolsäure und O-n-Valerylglykolsäure.

Als anorganisches Säurehalogenid verwendet man ein anorganisches Säurechlorid oder Säurebromid. Geeignet als anorganisches Säurehalogenid sind beispielsweise Phosphortrichlorid, Phosphorpentachlorid, Thionylchlorid oder Phosphortribromid, insbesondere Phosphortrichlorid oder Thionylchlorid, bevorzugt Phosphortrichlorid.

Die Umsetzung läßt sich in Abwesenheit oder Anwesenheit eines Lösungsmittels durchführen. Das verwendete Lösungsmittel soll sich unter den Bedingungen der Umsetzung inert verhalten. Als inertes organisches Lösungsmittel eignen sich beispielsweise Benzol, Toluol, o-Xylol, m-Xylol, p-Xylol, ein Gemisch isomerer Xylole, Chlorbenzol, Dichlorbenzol, Chlortoluol oder Diethylether, insbesondere Toluol, o-Xylol, m-Xylol, p-Xylol oder ein Gemisch isomerer Xylole.

Wie bereits dargelegt, führt man die Umsetzung üblicherweise bei 20 bis 180°C aus. In einer Vielzahl von Fällen hat es sich bewährt, die Umsetzung bei 60 bis 160°C, insbesondere 90 bis 150°C ablaufen zu lassen.

An die Reihenfolge, mit der die drei Reaktionsteilnehmer, nämlich das Anilin der Formel (2), die O-Acylglykolsäure der Formel (3) und das anorganische Säurehalogenid, der Umsetzung zugeführt werden, sind keine besonderen Anforderungen gestellt. Die Reaktionsteilnehmer können in beliebiger Reihenfolge der Umsetzung zugeleitet werden.

Besonders einfach gestaltet sich das Verfahren, indem man das Anilin der Formel (2) und die O-Acylglykolsäure der Formel (3), gegebenenfalls zusammen mit inertem organischen Lösungsmittel, vorlegt und das anorganische Säurehalogenid, gegebenenfalls zusammen mit inertem organischen Lösungsmittel, zudosiert. Es ist selbstverständlich, daß generell für eine gute Vermischung der Reaktanden zu sorgen ist.

Im allgemeinen setzt man das Anilin der Formel (2), die Acylglykolsäure der Formel (3) und das anorganische Säurehalogenid im Molverhältnis, respektive im Verhältnis ihrer Äquivalente, von 1 : (0,5 bis 1,5) : (0,2 bis 3), insbesondere 1 : (0,75 bis 1,25): (0,5 bis 2), bevorzugt 1 : (0,9 bis 1,1) : (0,8 bis 1,3) ein.

Das erfindungsgemäße Verfahren läßt sich unter reduziertem Druck, unter Atmosphärendruck oder unter erhöhtem Druck durchführen. Es läßt sich besonders einfach unter Atmosphärendruck durchführen. In einigen Fällen kann es von Vorteil sein, die Reaktion unter erhöhtem Druck, beispielsweise unter dem sich jeweils einstellenden Reaktionsdruck, ablaufen zu lassen. Das Verfahren läßt sich kontinuierlich oder diskontinuierlich durchführen. Besonders einfach gestaltet sich eine diskontinuierliche Durchführung des Verfahrens.

Die nachfolgenden Beispiele belegen die Erfindung, ohne sie zu beschränken.

### Experimenteller Teil

### Beispiel 1

### Herstellung von Acetoxyessigsäure-N-isopropyl-(4-fluoranilid)

In einem Reaktionskolben werden 11,8 g (0,1 mol) Acetoxyessigsäure und 16,8 g (0,11 mol) 4-Fluor-N-isopropylanilin in 100 ml Toluol vorgelegt. Bei Raumtemperatur wird unter Rühren eine Lösung von 5,1 g (0,037 mol) Phosphortrichlorid in 30 ml Toluol zugetropft. Anschließend wird der Kolbeninhalt unter Rückfluß gekocht. Dabei wird der Verlauf der Reaktion gaschromatographisch verfolgt. Nach 4 Stunden läßt man auf Raumtemperatur abkühlen und tropft eine Lösung von 3 g (0,022 mol) Phosphortrichlorid in 10 ml Toluol zu. Die Reaktionsmischung wird nochmals 8 Stunden unter Rückfluß gekocht. Man schüttelt die Toluolphase mit Wasser aus und entfernt das Lösungsmittel im Vakuum. Der Rückstand wird durch Behandlung mit Petrolether zur Kristallisation gebracht. Man erhält 16,5 g (65,1 % d. Th.) Acetoxyessigsäure-N-isopropyl-(4-fluoranilid) mit einer Reinheit von 98,8 % (GC).

### Beispiel 2

### Herstellung von Acetoxyessigsäure-N-isopropyl-(4-fluoranilid)

In einem Reaktionskolben werden 23,6 g (0,2 mol) Acetoxyessigsäure und 30,6 g (0,2 mol) 4-Fluor-N-isopropylanilin in 200 ml Toluol vorgelegt. Bei Raumtemperatur wird unter Rühren eine Lösung von 26,2 g (0,22 mol) Thionylchlorid in 40 ml Toluol zugetropft. Anschließend wird der Kolbeninhalt auf 100-110° C erwärmt. Der Verlauf der Reaktion wird gaschromatographisch verfolgt. Nach 2 Stunden läßt man auf Raumtemperatur abkühlen und wäscht das Reaktionsgemisch mit Wasser. Dann destilliert man das Lösungsmittel im Vakuum ab und bringt den Rückstand durch Behandlung mit Petrolether zur Kristallisation. Man erhält 37,3 g (73,7 % d. Th.) Acetoxyessigsäure-N-isopropyl-(4-fluoranilid) mit einer Reinheit von 98,5 % (GC).

### Beispiel 3

### Herstellung von 2-Methoxy-(α-acetoxyessigsäure)-anilid

In einem Reaktionskolben werden 23,6 g (0,2 mol) Acetoxyessigsäure und 24,6 g (0,2 mol) o-Anisidin in 200 ml Toluol vorgelegt. Bei Raumtemperatur wird unter Rühren eine Lösung von 26,2 g (0,22 mol) Thionylchlorid in 40 ml Toluol zugetropft. Anschließend wird der Kolbeninhalt auf 100-110° C erwärmt. Der Verlauf der Reaktion wird gaschromatographisch verfolgt. Nach 1 Stunde läßt man auf Raumtemperatur abkühlen und wäscht das Reaktionsgemisch mit Wasser. Dann destilliert man das Lösungsmittel im Vakuum ab und bringt den Rückstand durch Behandlung mit Petrolether zur Kristallisation. Man erhält 29,8 g (62,6 % d. Th.) 2-Methoxy-(α-acetoxyessigsäure)-anilid mit einer Reinheit von 91,4 % (GC).

## Patentansprüche

1. Verfahren zur Herstellung von O-Acylglykolsäureaniliden der allgemeinen Formel (1) 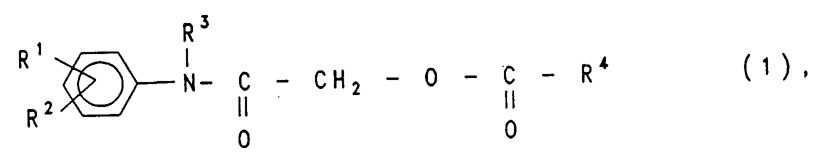 worin R¹ und R² gleich oder verschieden sind und für Wasserstoff, Halogen, eine Nitro-, Cyano-, eine geradkettige oder verzweigte Alkyl-, Alkenyl-, Alkinyl-, Alkoxy-Gruppe mit 1 bis 12 Kohlenstoffatomen, eine Aralkyl-Gruppe mit 7 bis 12 Kohlenstoffatomen, eine Cycloalkyl-Gruppe mit 6 bis 12 Kohlenstoffatomen oder eine Aryl-Gruppe mit 6 bis 12 Kohlenstoffatomen stehen, R³ Wasserstoff oder eine geradkettige oder verzweigte Alkyl-Gruppe mit 1 bis 12 Kohlenstoffatomen bedeutet und R⁴ für eine Alkyl-Gruppe mit 1 bis 6 Kohlenstoffatomen oder einen substituierten oder unsubstituierten Phenylrest steht, dadurch gekennzeichnet, daß man ein Anilin der allgemeinen Formel (2) 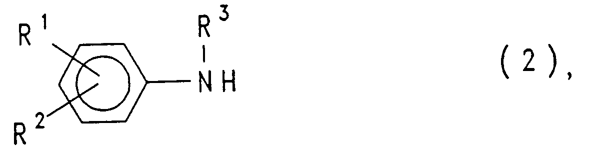 worin R¹, R² und R³ dieselbe Bedeutung wie in Formel (1) haben, und eine O-Acylglykolsäure der allgemeinen Formel (3) 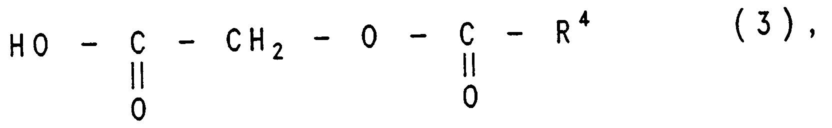 worin R⁴ dieselbe Bedeutung wie in Formel (1) hat, mit einem anorganischen Säurehalogenid, gegebenenfalls in Anwesenheit eines inerten organischen Lösungsmittels, bei 20 bis 180° C umsetzt.

2. Verfahren nach Anspruch 1, dadurch gekennzeichnet, daß man ein Anilin der Formel (2), worin R¹ und R² gleich oder verschieden sind und für Wasserstoff, Halogen, eine Nitro-, eine geradkettige oder verzweigte Alkyl-, Alkoxy-Gruppe mit 1 bis 4 Kohlenstoffatomen, oder eine Aralkyl-Gruppe mit 7 bis 12 Kohlenstoffatomen stehen, einsetzt.

3. Verfahren nach Anspruch 1 oder 2, dadurch gekennzeichnet, daß man ein Anilin der Formel (2), worin R¹ und R² gleich oder verschieden sind und für Wasserstoff, Fluor, Chlor, Brom, eine Alkyl- oder Alkoxy-Gruppe mit 1 bis 4 Kohlenstoffatomen stehen, einsetzt.

4. Verfahren nach einem oder mehreren der Ansprüche 1 bis 3, dadurch gekennzeichnet, daß man ein Anilin der Formel (2), worin R¹ und R² gleich oder verschieden sind und für Wasserstoff, Fluor, Chlor, Brom oder eine Alkylgruppe mit 1 bis 4 Kohlenstoffatomen stehen, einsetzt.

5. Verfahren nach einem oder mehreren der Ansprüche 1 bis 4, dadurch gekennzeichnet, daß man ein Anilin der Formel (2), worin R³ für Wasserstoff oder eine geradkettige oder verzweigte Alkyl-Gruppe mit 1 bis 4 Kohlenstoffatomen steht, einsetzt.

6. Verfahren nach einem oder mehreren der Ansprüche 1 bis 5, dadurch gekennzeichnet, daß man ein Anilin der Formel (2), worin R³ für eine Isopropyl-Gruppe steht, einsetzt.

7. Verfahren nach einem oder mehreren der Ansprüche 1 bis 6, dadurch gekennzeichnet, daß man eine O-Acylglykolsäure der Formel (3), worin R⁴ für eine Methyl-, Ethyl-, n-Propyl, i-Propyl-Gruppe oder einen Phenylrest steht, einsetzt.

8. Verfahren nach einem oder mehreren der Ansprüche 1 bis 7, dadurch gekennzeichnet, daß man eine O-Acylglykolsäure der Formel (3), worin R⁴ für eine Methyl-Gruppe steht, einsetzt.

9. Verfahren nach einem oder mehreren der Ansprüche 1 bis 8, dadurch gekennzeichnet, daß man als anorganisches Säurehalogenid ein anorganisches Säurechlorid oder Säurebromid einsetzt.

10. Verfahren nach einem oder mehreren der Ansprüche 1 bis 9, dadurch gekennzeichnet, daß man als anorganisches Säurehalogenid Phosphortrichlorid, Phosphorpentachlorid, Thionylchlorid, Phosphortribromid einsetzt.

11. Verfahren nach einem oder mehreren der Ansprüche 1 bis 10, dadurch gekennzeichnet, daß man als anorganisches Säurehalogenid Phosphortrichlorid oder Thionylchlorid einsetzt.

12. Verfahren nach einem oder mehreren der Ansprüche 1 bis 11, dadurch gekennzeichnet, daß man als inertes organisches Lösungsmittel Benzol, Toluol, o-Xylol, m-Xylol, p-Xylol, ein Gemisch isomerer Xylole, Chlorbenzol, Dichlorbenzol, Chlortoluol oder Diethylether einsetzt.

13. Verfahren nach einem oder mehreren der Ansprüche 1 bis 12, dadurch gekennzeichnet, daß man als inertes organisches Lösungsmittel Toluol, o-Xylol, m-Xylol, p-Xylol oder ein Gemisch isomerer Xylole einsetzt.

14. Verfahren nach einem oder mehreren der Ansprüche 1 bis 13, dadurch gekennzeichnet, daß man die Umsetzung bei 60 bis 160°C durchführt.

15. Verfahren nach einem oder mehreren der Ansprüche 1 bis 14, dadurch gekennzeichnet, daß man die Umsetzung bei 90 bis 150°C durchführt.

16. Verfahren nach einem oder mehreren der Ansprüche 1 bis 15, dadurch gekennzeichnet, daß man das Anilin der Formel (2), die O-Acylglykolsäure der Formel (3) und das anorganische Säurehalogenid im Molverhältnis 1 : (0,5 bis 1,5) : (0,2 bis 3) einsetzt.

## Claims

1. A process for the preparation of O-acylglycolanilides of the formula (1) 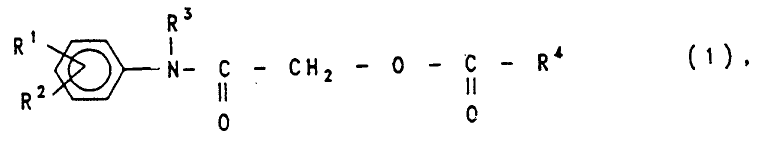 in which R¹ and R² are identical or different and are hydrogen, halogen, a nitro group, a cyano group, a straight-chain or branched alkyl, alkenyl, alkynyl or alkoxy group having from 1 to 12 carbon atoms, an aralkyl group having from 7 to 12 carbon atoms, a cycloalkyl group having from 6 to 12 carbon atoms or an aryl group having from 6 to 12 carbon atoms, R³ is hydrogen or a straight-chain or branched alkyl group having from 1 to 12 carbon atoms, and R⁴ is an alkyl group having from 1 to 6 carbon atoms or is a substituted or unsubstituted phenyl radical, which comprises reacting an aniline of the formula (2) 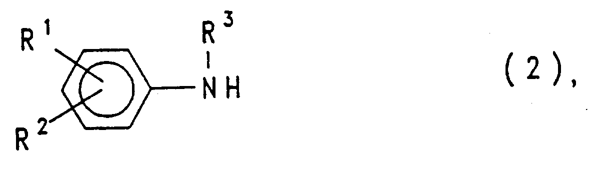 in which R¹, R² and R³ have the same meaning as in formula (1), and an O-acylglycolic acid of the formula (3) 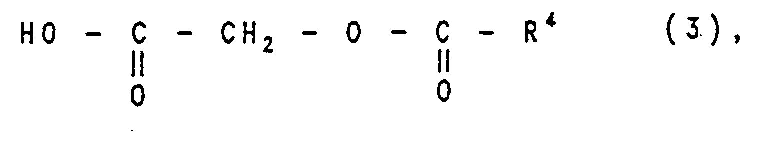 in which R⁴ has the same meaning as in formula (1), with an inorganic acid halide, optionally in the presence of an inert organic solvent, at from 20 to 180°C.

2. The process as claimed in claim 1, wherein an aniline of the formula (2) is employed in which R¹ and R² are identical or different and are hydrogen, halogen, a nitro group, a straight-chain or branched alkyl or alkoxy group having from 1 to 4 carbon atoms, or an aralkyl group having from 7 to 12 carbon atoms.

3. The process as claimed in claim 1 or 2, wherein an aniline of the formula (2) is employed in which R¹ and R² are identical or different and are hydrogen, fluorine, chlorine, bromine, or an alkyl or alkoxy group having from 1 to 4 carbon atoms.

4. The process as claimed in one or more of claims 1 to 3, wherein an aniline of the formula (2) is employed in which R¹ and R² are identical or different and are hydrogen, fluorine, chlorine, bromine or an alkyl group having from 1 to 4 carbon atoms.

5. The process as claimed in one or more of claims 1 to 4, wherein an aniline of the formula (2) is employed in which R³ is hydrogen or a straight-chain or branched alkyl group having from 1 to 4 carbon atoms.

6. The process as claimed in one or more of claims 1 to 5, wherein an aniline of the formula (2) is employed in which R³ is an isopropyl group.

7. The process as claimed in one or more of claims 1 to 6, wherein an O-acylglycolic acid of the formula (3) is employed in which R⁴ is a methyl, ethyl, n-propyl or i-propyl group or a phenyl radical.

8. The process as claimed in one or more of claims 1 to 7, wherein an O-acylglycolic acid of the formula (3) is employed in which R⁴ is a methyl group.

9. The process as claimed in one or more of claims 1 to 8, wherein an inorganic acid chloride or acidbromide is employed as the inorganic acid halide.

10. The process as claimed in one or more of claims 1 to 9, wherein phosphorus trichloride, phosphorus pentachloride, thionyl chloride or phosphorus tribromide is employed as the inorganic acid halide.

11. The process as claimed in one or more of claims 1 to 10, wherein phosphorus trichloride or thionyl chloride is employed as the inorganic acid halide.

12. The process as claimed in one or more of claims 1 to 11, wherein benzene, toluene, o-xylene, m-xylene, p-xylene, a mixture of isomeric xylenes, chlorobenzene, dichlorobenzene, chlorotoluene or diethyl ether is employed as the inert organic solvent.

13. The process as claimed in one or more of claims 1 to 12, wherein toluene, o-xylene, m-xylene, p-xylene or a mixture of isomeric xylenes is employed as the inert organic solvent.

14. The process as claimed in one or more of claims 1 to 13, wherein the reaction is carried out at from 60 to 160°C.

15. The process as claimed in one or more of claims 1 to 14, wherein the reaction is carried out at from 90 to 150°C.

16. The process as claimed in one or more of claims 1 to 15, wherein the aniline of the formula (2), the O-acylglycolic acid of the formula (3) and the inorganic acid halide are employed in a molar ratio of 1 : (0.5 to 1.5) : (0.2 to 3).

## Revendications

1. Procédé pour la préparation d'anilides de l'acide O-acyl-glycolique de formule générale (1) 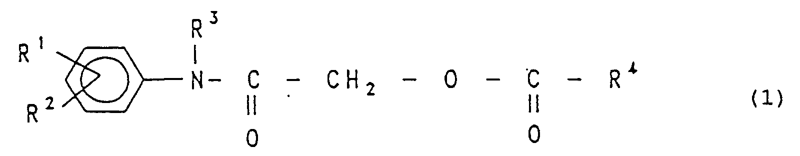 dans laquelle R¹ et R² sont identiques ou différents et représentent l'hydrogène, halogène, un groupe nitro, cyano, alkyle, alcényle, alcynyle, alcoxy linéaire ou ramifié avec 1 à 12 atomes de carbone, un groupe aralkyle avec 7 à 12 atomes de carbone, un groupe cycloalkyle avec 6 à 12 atomes de carbone ou un groupe aryle avec 6 à 12 atomes de carbone, R³ représente l'hydrogène ou un groupe alkyle linéaire ou ramifié avec 1 à 12 atomes de carbone et R⁴ représente un groupe alkyle avec 1 à 6 atomes de carbone ou un radical phényle substitué ou non substitué, caractérisé en ce que l'on fait réagir une aniline de formule générale (2) 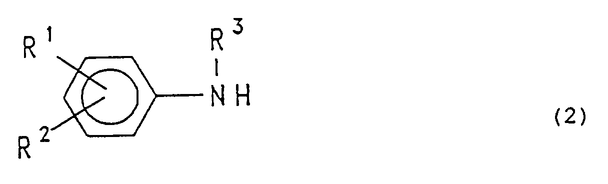 dans laquelle R¹, R² et R³ ont la même signification que dans la formule (1) et un acide O-acyl-glycolique de formule générale (3) 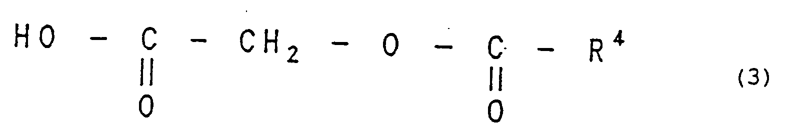 dans laquelle R⁴ a la même signification que dans la formule (1), avec un halogénure d'acide inorganique, éventuellement en présence d'un solvant organique inerte, à une température de 20 à 180 °C.

2. Procédé selon la revendication 1, caractérisé en ce que l'on utilise une aniline de formule (2), dans laquelle R¹ et R² sont identiques ou différents et représentent l'hydrogène, halogène, un groupe nitro, un groupe alcoxy, un groupe alkyle linéaire ou ramifié avec 1 à 4 atomes de carbone, ou un groupe aralkyle avec 7 à 12 atomes de carbone.

3. Procédé selon la revendication 1 ou 2, caractérisé en ce que l'on utilise une aniline de formule (2), dans laquelle R¹ et R² sont identiques ou différents et représentent l'hydrogène, le fluor, le chlore, le brome, un groupe alkyle ou alcoxy avec 1 à 4 atomes de carbone.

4. Procédé selon une ou plusieurs des revendications 1 à 3, caractérisé en ce que l'on utilise une aniline de formule (2), dans laquelle R¹ et R² sont identiques ou différents et représentent l'hydrogène, le fluor, le chlore, le brome ou un groupe alkyle avec 1 à 4 atomes de carbone.

5. Procédé selon une ou plusieurs des revendications 1 à 4, caractérisé en ce que l'on utilise une aniline de formule (2) dans laquelle R³ représente l'hydrogène ou un groupe alkyle linéaire ou ramifié avec 1 à 4 atomes de carbone.

6. Procédé selon une ou plusieurs des revendications 1 à 5, caractérisé en ce que l'on utilise une aniline de formule (2) dans laquelle R³ représente un groupe isopropyle.

7. Procédé selon une ou plusieurs des revendications 1 à 6, caractérisé en ce que l'on utilise un acide O-acyl-glycolique de formule (3), dans laquelle R⁴ représente un groupe méthyle, éthyle, n-propyle, i-propyle ou un radical phényle.

8. Procédé selon une ou plusieurs des revendications 1 à 7, caractérisé en ce que l'on utilise un acide O-acyl-glycolique de formule (3), dans laquelle R⁴ représente un groupe méthyle.

9. Procédé selon une ou plusieurs des revendications 1 à 8, caractérisé en ce que l'on utilise comme halogénure d'acide inorganique un chlorure d'acide ou bromure d'acide inorganique.

10. Procédé selon une ou plusieurs des revendications 1 à 9, caractérisé en ce que l'on utilise comme halogénure d'acide inorganique le trichlorure de phosphore, le pentachlorure de phosphore, le chlorure de thionyle, le tribromure de phosphore.

11. Procédé selon une ou plusieurs des revendications 1 à 10, caractérisé en ce que l'on utilise comme halogénure d'acide inorganique le trichlorure de phosphore ou le chlorure de thionyle.

12. Procédé selon une ou plusieurs des revendications 1 à 11, caractérisé en ce que l'on utilise en tant que solvant organique inerte le benzène, le toluène, le o-xylène, le m-xylène, le p-xylène, un mélange de xylènes isomères, le chlorobenzène, le dichlorobenzène, le chlorotoluène ou l'éther diéthylique.

13. Procédé selon une ou plusieurs des revendications 1 à 12, caractérisé en ce que l'on utilise comme solvants organiques inertes le toluène, l'o-xylène, le m-xylène, le p-xylène ou un mélange de xylènes isomères.

14. Procédé selon une ou plusieurs des revendications 1 à 13, caractérisé en ce que l'on met en oeuvre la réaction à une température de 60 à 160 °C.

15. Procédé selon une ou plusieurs des revendications 1 à 14, caractérisé en ce que l'on met en oeuvre la réaction à une température de 90 à 150 °C.

16. Procédé selon une ou plusieurs des revendications 1 à 15, caractérisé en ce que l'on utilise l'aniline de formule (2), l'acide O-acyl-glycolique de formule (3) et l'halogénure d'acide inorganique dans un rapport en moles de 1:(0,5 à 1,5):(0,2 à 3).
